# EUROPEAN PATENT SPECIFICATION

(11) **EP 1 807 083 B1**
(45) Date of publication and mention of the grant of the patent: **30.03.2011**
(21) Application number: 05798339.7
(22) Date of filing: 02.11.2005
(51) Int. Cl.: A61K 31/498, A61P 17/06, A61P 17/00

(54) **USE OF PIRLINDOLE FOR THE TREATMENT OF DISEASES WHICH ARE CHARACTERIZED BY PROLIFERATION OF T-LYMPHOCYTES AND/OR HYPERPROLIFERATION OF KERATINOCYTES IN PARTICULAR ATOPIC DERMATITIS AND PSORIASIS**
VERWENDUNG VON PIRLINDOL BEI DER BEHANDLUNG VON ERKRANKUNGEN; DIE DURCH PROLIFERATION VON T-LYMPHOZYTEN UND/ODER HYPERPROLIFERATION VON KERATINOZYTEN GEKENNZEICHNET SIND; INSBESONDERE ATOPISCHE DERMATITIS UND PSORIASIS
UTILISATION DE PIRLINDOLE POUR TRAITER DES MALADIES CARACTERISEES PAR UNE PROLIFERATION DE LYMPHOCYTES T ET/OU UNE HYPERPROLIFERATION DE KERATINOCYTES, EN PARTICULIER UNE DERMATITE ATOPIQUE ET UN PSORIASIS

(30) Priority: 02.11.2004 EP 04025961
(43) Date of publication of application: 18.07.2007
(73) Proprietor: Switch Biotech, LLC, Conway, AR 72034 (US)
(72) Inventor: BELL, Stefan, 76530 Baden-Baden (DE)
(74) Representative: Zwicker, Jörk
(86) International application number: PCT/EP2005/011697
(87) International publication number: WO 2006/048242

(56) References cited:
- EP-A- 0 626 385
- EP-A- 1 688 161
- US-A1- 2004 142 904
- US-A1- 2004 167 201
- EGOROVA E F ET AL: "MECHANISM OF PYRAZIDOL POTENTIATION OF THE HYPER GLYCEMIC EFFECT OF EPINEPHRINE" FARMAKOLOGIYA I TOKSIKOLOGIYA, MEDICINA, MOSCOW, RU, vol. 41, no. 1, 1978, pages 36-40, XP009044719 ISSN: 0014-8318
- LIBERMAN S S ET AL: "THE EFFECT OF PYRAZIDOLE ON BLOOD SUGAR CONCENTRATION CHANGES CAUSED BY ADRENALINE AND SEROTONIN IN INTACT AND ADRENALECTOMIZED ANIMALS" FARMAKOLOGIYA I TOKSIKOLOGIYA, MEDICINA, MOSCOW, RU, vol. 40, no. 4, 1977, pages 442-445, XP009044725 ISSN: 0014-8318

## Description

The present invention relates to the use of Pirlindole if needed with appropriate additives and auxiliary substances for the production of a medicament for the treatment of diseases characterized by proliferation of T-lymphocytes and/or the hyperproliferation of keratinocytes in particular psoriasis and atopic dermatitis as well as compositions comprising Pirlindole and their use.

### Prior Art

Pirlindole (2, 3, 3a, 4, 5, 6-hexahydro-8-methyl-1H-pyrazino[3,2,1-jk]carbazole) of the following formula: 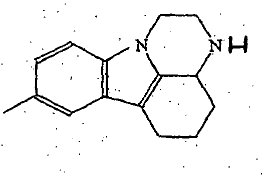 is a tetracyclic compound that has been characterized as an antidepressant drug (Tanghe A, Geerts S, Van Dorpe J et al., Acta Psychiatr Scand 96/2: 134-141, 1997; Bruhwyler J, J liégeois JF, Gérardy J & al, Behav Pharmacol 9: 731-737, 1998; Ginsberg F, Joos E, Greczy J & al, J Neuroskelet Pain 6/2: 5-17, 1998). On a molecular level the mechanism of action has not been completely elucidated. A proposed mechanism of action consists of a selective and reversible inhibition of monoamine oxidase A (De Wilde J, Mertens C, Van Dorpe J et al., Hum Psychopharmacol: 12/1: 41-46, 1997; Medvedev AE, Shvedov VI, Chulkova TM et al., Neurochem Res 21/12: 1521-1526, 1996.). Secondarily, it exerts an inhibitory effect on noradrenaline and 5-hydroxytryptamine reuptakes. It has no effect on the dopaminergic and cholinergic, systems. It has only a low potential for amplifying tyramine and noradrenaline repressor effect.

US 2004/142904 A1 discloses that Pirlindole can be administered adjunctively with Milnacipran and that Milnacipran derivatives are indicated for use in the treatment of autoimmune diseases, e.g. psoriasis.

US 2004/167201 A1 discloses that Melanocortin 1 receptor (MC1-R)-specific compounds which are pyrrolidin-derivatives can be used for the treatment of inflammatory diseases, e.g. psoriasis. This document further discloses that pirlindole may be used with pyrrolidin-derivatives for the treatment of weight and feeding-related disorders.

Pirlindole has an absolute bioavailability of between 20 and 30% due to an expensive first-pass effect. Acute and chronic toxicological studies have not revealed potentially dangerous effects of the drug at the usual doses. It does not present measurable mutagenic, clastogenic or carcinogenic properties. (Bruhwyler J, Liegeois JF, Geczy J, Pharmacol Res. 1997 Jul;36(1):23-33.)

### Invention

It was now surprisingly found that Pirlindole can successfully inhibit proliferation of keratinocytes and/or T-lymphocytes (T cells) and therefore is surprisingly suited if desired in combination with appropriate adjuvants and additives to treat and/or to prevent the onset of diseases characterized by hyperproliferation of keratinocytes and/or T cells. Examples of such diseases are psoriasis in particular psoriasis vulgaris, psoriasis capitis, psoriasis guttata, psoriasis inversa, atopic dermatitis, actinic keratosis, hyperkeratosis like epidermolytic hyperkeratosis, hyperkeratosis lenticularis perstans as well as keratosis pilaris, ichthyoses, alopecia areata, alopecia totalis, alopecia subtotalis, alopecia universalis, alopecia diffusa, lupus erythematodes of the skin, lichen planus, dermatomyostis of the skin, atopic eczema, morphea, scleroderma, alopecia areata Ophiasis type, androgenic alopecia, allergic contact dermatitis, irritative contact dermatitis, contact dermatitis, pemphigus vulgaris, pemphigus foliaceus, pemphigus vegetans, scarring mucous membrane pemphigoid, bullous pemphigoid, mucous membrane pemphigoid, dermatitis, dermatitis herpetiformis Duhring, urticaria, necrobiosis lipoidica, erythema nodosum, lichen vidal, prurigo simplex, prurigo nodularis, prurigo acuta, linear IgA dermatosis, polymorphic light dermatosis, erythema solaris, lichen sclerosus et atrophicans, exanthema of the skin, drug exanthema, purpura chronica progressiva, dihidrotic eczema, eczema, fixed drug exanthema, photoallergic skin reaction, lichen simplex periorale dermatitis, graft-versus-host-disease, acne, rosacea, abnormal scarring, keloids and vitiligo. Preferred diseases are atopic dermatitis and psoriasis, in particular psoriasis.

Therefore, the invention relates to the use of Pirlindole or of a pharmaceutically acceptable salt thereof for the production of a medicament for the therapy or prevention of diseases which are characterized by hyperproliferation of keratinocytes and/or T cells. If needed Pirlindole or a pharmaceutically acceptable salt thereof can be combined with suitable adjuvants and additives.

In a preferred embodiment the disease is selected from psoriasis, atopic dermatitis, actinic keratosis, hyperkeratosis like epidermolytic hyperkeratosis, hyperkeratosis lenticularis perstans, keratosis pilaris and ichthyoses.

Particularly preferred diseases are psoriasis and atopic dermatitis, in particular psoriasis.

### Diseases that a characterized by hyperproliferation of keratinocytes and/or T cells

Diseases which are characterized by hyperproliferation of keratinocytes within the meaning of the present invention are diseases wherein patients exhibit locally or over the whole body a thickened epidermis in comparison to healthy epidermis. A thickened epidermis is deemed to be an epidermis, which is thickened in comparison to healthy skin by at least about 10%, preferably about 30%, in particular about 50% and most preferably about 80%. Methods for measuring thickness of epidermis are known to someone skilled in the art. Wetzel et al. (Arch. Dermatol. Res., April 2003) describe, for example, optical coherence tomography and Baulieu et al. (Proc. Natl. Acad. Sci. USA, 2000, 97:4279-4284), skin echographic measurement, which both represent non-invasive methods for the measurement of the thickness of the epidermis. Furthermore the thickness of the epidermis can be determined histologically in section of skin biopsies as described in, for example, El-Domyati et al., (Exp. Dermatol., 2002; 11:398-405) or Schopf et al. (J. Am. Acad. Dermatol. 2002; 46:886-91.). Since the epidermis exhibits different thickness in different regions of the skin it is necessary for a comparison of the thickness of healthy and diseased epidermis to compare the respective thickness of the epidermis in similar regions of the skin. Furthermore there is a certain variation of the thickness of the epidermis within the same regions of the skin among two individuals. It is therefore preferred that the thickness of the epidermis is measured, for example, at the left and at the right leg of a diseased individual under the precondition that not the complete skin is affected by the disease. In general diseases characterized by hyperproliferation of keratinocytes are accompanied by a reddening of the effected region of the skin such that someone skilled in the art can distinguish diseased regions of the skin of the patients from healthy regions of the skin solely based on the reddening. The thickening of epidermis in diseases characterized by hyperproliferation of keratinocytes can occur, for example, only locally or can already be detectable, as in psoriasis, in the skin of psoriasis patients which is not discernibly effected based on a reddening and a lesion, respectively. In psoriasis patients a further thickening of the epidermis is, however, also detectable in effected areas of the skin (=lesion). Examples of diseases, which are characterized by hyperproliferation of keratinocytes within the meaning of the present invention are psoriasis, in particular psoriasis vulgaris, psoriasis capitis, psoriasis guttata, psoriasis inversa, atopic dermatitis, actinic keratosis, hyperkeratosis with epidermolytic hyperkeratosis and hyperkeratosis lenticularis perstans as well as keratosis pilaris, acne, abnormal scarring, keloids and ichthyoses. Particularly preferred diseases within the meaning of the present invention are atopic dermatitis and psoriasis, in particular psoriasis.

Epidermis is primarily formed from keratinocytes which slowly migrate from basal membrane to the exterior. During this process they pass from a proliferating into a differentiated status to finally die off. Then the dead keratinocytes form the subcorneous at the surface of the skin, which constantly sheds dead cells. By this process a constant regeneration of the skin is achieved. In diseases, which are characterized by hyperproliferation of keratinocytes the balance between differentiation and proliferation of keratinocytes is tilted towards proliferation whereby the epidermis, which comprises more keratinocytes, in particular proliferating keratinocytes is significantly thickened. In such diseases distorted barrier functions are also often found whereby superantigens or pathogens can penetrate the skin more easily. Often an increased inflammation is also observed as e.g. with atopic dermatitis and psoriasis which is then accompanied by the reddening of the skin already mentioned.

Surprisingly it has been observed within the context of the present invention that Pirlindole also has an inhibiting effect on the hyperproliferation of T cells. This further effect increases on one hand the effectiveness of Pirlindole and compositions comprising Pirlindole for diseases wherein the disease pattern is characterized both by a hyperproliferation of keratinocytes and a hyperproliferation of T cells and on the other hand opens up the possibility to use Pirlindole for diseases which are primarily characterized by hyperproliferation of T cells.

Diseases characterized by hyperproliferation of T cells within the meaning of the present invention are diseases in which the patients locally or over the whole body, primarily in the skin exhibit an increased number of proliferating T cells in comparison to healthy regions of the body, in particular to healthy skin. The number of proliferating T cells is deemed increased, if the region of the body, in particular region of the skin examined comprises at least about 10% preferably at least about 30%, in particular about 50% more preferably 100%, most preferably 200% or more proliferating T cells. The term "region of the body" as used herein can comprise any region and organ, respectively, like, e.g. skin, hematopoietic system and lymph nodes. The term "skin" comprises epidermis, dermis and subcutis, however, in particular the epidermis. The number of proliferating T cells can be determined by a variety of methods known in the prior art. The number of T cells in S or G₂ phase can be determined by, e.g. histological staining of a skin punch biopsy or a single cell suspension obtained, from a skin punch biopsy, can be examined by FACS analysis for the cell cycle phases of the cells.

Examples of diseases that are characterized by hyperproliferation of T cells within the meaning of the present invention are psoriasis, atopic dermatitis, alopecia areata, alopecia totalis, alopecia subtotalis, alopecia universalis, alopecia diffusa,lupus erythematodes of the skin, lichen planus, dermatomyositis of the skin, atopic eczema, morphea, scleroderma, psoriasis vulgaris, psoriasis capitis, psoriasis guttata, psoriasis inversa, alopecia areata Ophiasis type, androgenic alopecia, allergic contact dermatitis, irritative contact dermatitis, contact dermatitis, pemphigus vulgaris, pemphigus foliaceus, pemphigus vegetans, scarring mucous membrane pemphigoid, bullous pemphigoid, mucous membrane pemphigoid, dermatitis, dermatitis herpetiformis Duhring, urticaria, necrobiosis lipoidica, erythema nodosum, lichen vidal, prurigo simplex, prurigo nodularis, prurigo acuta, linear IgA dermatosis, polymorphic light dermatosis, erythema solaris, lichen sclerosus et atrophicans, exanthema of the skin, drug exanthema, purpura chronica progressiva, dihidrotic eczema, eczema, fixed drug exanthema, photoallergic skin reaction, lichen simplex periorale dermatitis, rosacea, and vitiligo.

In particular psoriasis and atopic dermatitis are diseases which are both characterized by hyperproliferation of keratinocytes and of T cells and Pirlindole and Pirlindole-comprising compositions are particularly suitable for the therapy thereof since they attack the diseases by at least two different modes of action.

Presently only unsatisfactory therapies for the treatment of these diseases exist, which are often only effective in patient subpopulations and existing therapies like topic or systemic application of corticosteriods or cyclosporine in the case of atopic dermatitis or psoriasis are often accompanied by severe adverse effects. There is, therefore, a need for new medicaments preferably without adverse effects for the therapy of these diseases. Pirlindole useable according to the present invention is one such medicament. In addition Pirlindole is suitable for topic application because of its lipophilicity thereby allowing to further reduce adverse effects.

Medicaments useable according to the present invention can be used for the treatment of local lesions but also for the prevention of the onset of the disease. Thus, it is possible to prevent the onset of the disease with dermatological manifestation by early treatment of psoriasis patients without lesions, for example, by inhibiting the further thickening of the epidermis by administration of Pirlindole.

### Pharmaceutically acceptable salts

Pirlindole useable according to the present invention can be provided in any number of forms suitable for administration. Suitable pharmaceutically acceptable forms comprise salts or pre or pro-forms of Pirlindole.

Examples of pharmaceutically acceptable salts comprise without limitation non toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfate derived from p-toluene-sulfonic acid and others. Such salts can be produced by methods known to someone of skill in the art and described in the prior art.

Other salts like oxalate derived from oxalic acid, which is not considered as pharmaceutically acceptable can be appropriate as intermediates for the production of Pirlindole or a pharmaceutically acceptable salt thereof.

### Formulation

The term "adjuvant" according to the invention refers to any pharmaceutically acceptable solid or liquid filler, dilution or packaging material as long as it does not disadvantageously react with Pirlindole or a pharmaceutically acceptable salt thereof. Liquid galenic adjuvants are, for example, sterile water, physiological saline solution, sugar solution, ethanol and/or oils. Galenic adjuvants for the production of tablets and capsules can comprise, for example, binders and fillers.

The production of medicaments comprising Pirlindole and its application during the use according to the present invention is usually carried out according to established pharmaceutical technological methods. To this end Pirlindole is processed together with appropriate pharmaceutically acceptable adjuvants and carriers into the medicinal formulation, which is suitable for the different indications and the respective area of application. Thereby medicaments can be produced, which show the desired release rate, e.g. a quick flush and/or a retard and depot effect, respectively.

In a particularly preferred use of the present invention the above-described medicament is supplied topically for the therapy or prevention of diseases characterized by hyperproliferation of keratinocytes and/or T cells.

For the topical application onto skin, a wound or a mucous membrane the medicament comprising Pirlindole is preferably prepared in the form of an emulsion, a gel, an ointment, a foam, a band-aid, a cream of a mixed-phase and amphiphilic, respectively emulsion system (oil/water-water/oil-mixed-phase), a liposome or transferosome. These medicinal formulations are known in the prior art and the skilled practitioner can prepare Pirlindole without undue burden as a medicament having one of those medicinal formulations. In an especially preferred embodiment, the medicament is prepared in form of a cream, especially basis cream DAC (Deutsche Arzneimittel Codex) Basiscreme.

Further formulations, which can be topically applied are powders, pastes or solutions. Pastes often comprise as a base component lipophilic and hydrophilic additives with high solid content to provide consistency. The powders, in particular topically applied powders, can comprise for the increase the dispersity as well as the fluidity and the slideability as well as for the prevention of agglomerates, starches like wheat or rice starch, flame dispersion silicon dioxide and/or silica. These additives can also function as diluent.

In a preferred embodiment of the present invention the Pirlindole comprising medicament used for the therapy or prevention of a disease characterized by hyperproliferation of keratinocytes and/or T cells is therefore prepared as an ointment, a gel, a band-aid, an emulsion, a lotion, a foam, a cream of mixed-phase or amphiphilic emulsion systems (oil-water/water-oil mixed phase), a liposome, a transferosome, a paste, or a powder.

Particular suitable adjuvants and carriers, respectively, for the preparation of topically applied medicaments of the present invention are, for example, sodium alginate as gel-forming agent for the production of a suitable base or cellulose derivatives like, e.g. guar or xanthane gum, inorganic gel-forming agents like, e.g. aluminium hydroxide or betonite (so called thixotrope gel-forming agent), polyacrylic acid derivatives like, e.g. Carbopol^{®}, polyvinylpyrrolidone, microcrystalline cellulose or carboxymethyl cellulose, for example, the carboxymethyl cellulose product IntraSite (Smith & Nephew, London). Furthermore biocompatible polyoxameres can be used like, for example, FloGel^{®} which forms a thermoreversible gel. Furthermore phospholipids or amphiphilic low or high molecular weight compounds can be considered. The gels can either be hydrogels based on water or hydrophobic organogels, for example, on the basis of mixtures of lower and higher molecular weight paraffin carbohydrates and Vaseline. Further synthetic biomaterials can be employed as carriers whereby Pirlindole can be bound non-covalently or covalently, for example, directly or through a linker.

Skin soothing and/or anti-inflammation additives known to someone of skill in the art like, for example, synthetically produced substances and/or abstracts and/or substances from medicinal plants in particular bisobolol and panthenol can also be added to the medicament. Furthermore coloring agents like, for example, yellow and/or red ferrous oxide and/or titanium dioxide for the adjustment of color and/or fragrances can be added to the medicament.

In addition the medicaments usable according to the present invention can comprise emulsifying agents. Suitable emulsifying agents are neutral, anionic or cationic tensides, for example alkali soaps, metal soaps, amine soaps, sulfurated and sulfonated compounds, invert soaps, long-chain fatty alcohols, partial fatty acid ester of sorbitans and polyoxyethylene sorbitans, e.g. lanette-types, woolwax, lanoline or other synthetic products, which are suitable for the production of oil/water and/or water/oil emulsions. Hydrophilic organogels can be prepared, for example, on the basis of high molecular weight polyethylene glycols. This gel-type formulations are washable. Employed as lipids in the form of fatty and/or oily and/or waxy components for the preparation of ointments, crèmes or emulsions are Vaseline, natural and/or synthetic waxes, fatty acids, fatty alcohols, fatty acid esters, e.g. as mono-, di- or triglycerides, paraffin oils or vegetable oils, hardened castor oils or coconut oils, lard, synthetic fats, e.g. on the basis of caprylic, caprinic, lauric and stearic acid, like Softisan^{®} or mixtures of triglycerides like Miglyol^{®}.

To adjust pH values it is possible to use osmotically effective acids and bases, e.g. hydrochloric acid, citric acid, sodium hydroxide, potassium hydroxide, sodium hydrogen carbonate, further buffer-systems like, e.g. citrate, phosphate, Tris buffer, or triethanolamine. Furthermore the stability can be improved by the addition of preservatives like, e.g. methyl or propylene benzoate (parabene) or sorbic acid.

For nasal application nose drops, nasal spray atomizers or nasal creams or ointments can be employed. Nasal spray or dry powder preparations as well as aerosol dosage forms are suitable for the systemic administration of Pirlindole or a pharmaceutically acceptable salt thereof. Furthermore the medicaments according to the invention can be inhaled and insufflated by pressure and aerosol dosage forms, respectively, and dry powder formulations. Such formulations can also be used for the direct, regional application in the lung, the bronchial tubes and/or the larynx and for the local application, respectively. Dry powder formulations can be formulated, for example, as soft pellets of active agent, as powder mixtures of active-agent with suitable carriers like e.g. lactose and glucose. For the inhalation or insufflation commonly used devices known to someone of skill in the art can be employed, which are suitable for the treatment of the nasal, oral and/or pharyngeal cavity. Pirlindole or a pharmaceutically acceptable salt thereof can also be administered by means of an ultrasonic vaporizer. Instead of an aerosol dosage formulation it is also possible to use propellant-free manual pump systems. Suitably aerosols of propellants should comprise surfactant adjuvants like, e.g. isopropyl myristate, polyoxyethylene sorbitan, fatty acid ester, lecithin or soy lecithin. For the regional application *in situ,* for example, solutions for instillation are suitable.

Furthermore Pirlindole or a pharmaceutically acceptable salt thereof can be used in the form of systemically administered medicaments. These include parenterals, which comprise among others injectables and infusions. Injectables are formulated either in the form of ampoules or as so called ready-for-use injectables, e.g. ready-to-use syringes or single-use syringes and aside from this in puncturable flasks for multiple withdrawal. The administration of injectables can be in the form of subcutaneous (s.c.), intramuscular (i.m.), intravenous (i.v.) or intracutaneous (i.e.) application. In particular it is possible to produce the respectively suitable injection formulations as a suspension of crystals, solutions, nanoparticular or a colloid dispersed systems like, e.g. hydrosols.

Injectable formulations can further be produced as concentrates, which can be dissolved or dispersed with aqueous isotonic diluents. The infusion can also be prepared in form of isotonic solutions, fatty emulsions, liposomal formulations and micro emulsions. Similar to injectables infusion formulations can also be prepared in the form of concentrates for dilution. Injectable formulations can also be applied in the form of permanent infusions both in inpatient and ambulant therapy, e.g. by way of mini-pumps.

It is possible to add to parental drug formulations, for example, albumin, plasma, expander, surface-active substances, organic diluents, pH-influencing substances, complexing substances or polymeric substances, in particular as substances to influence the adsorption of Pirlindole or a pharmaceutically acceptable salt thereof to proteins or polymers or they can also be added with the aim to reduce the adsorption of Pirlindole or a pharmaceutically acceptable salt thereof to materials like injection instruments or packaging-materials, for example, plastic or glass.

Pirlindole or a pharmaceutically acceptable salt thereof can be bound to microcarriers or nanoparticles in parenterals like, for example, to finely dispersed particles based on poly(meth)acrylates, polylactates, polyglycolates, polyamino acids or polyether urethanes. Parenteral formulations can also be modified as depot preparations, e.g. based on the "multiple unit principle", if Pirlindole or a pharmaceutically acceptable salt thereof is introduced in finely dispersed, dispersed and suspended form, respectively, or as a suspension of crystals in the medicament or based on the "single unit principle" if Pirlindole or a pharmaceutically acceptable salt thereof is enclosed in a formulation, e.g. in a tablet or a rod which is subsequently implanted. These implants or depot medicaments in single unit and multiple unit formulations often consist of so called biodegradable polymers like e.g. polyesters of lactic and glycolic acid, polyether urethanes, polyamino acids, poly(meth)acrylates or polysaccharides.

Adjuvants and carriers added during the production of the medicaments usable according to the present invention formulated as parenterals are preferably aqua sterilisata (sterilized water), pH value influencing substances like, e.g. organic or inorganic acids or bases as well as salts thereof, buffering substances for adjusting pH values, substances for isotonization like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween^{®}) or, e.g. fatty acid esters of polyoxyethylenes (for example, Cremophor^{®}), fatty oils like, e.g. peanut oil, soybean oil or castor oil, synthetic esters of fatty acids like, e.g. ethyl oleate, isopropyl myristate and neutral oil (for example, Miglyol^{®}) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilizers like e.g. EDTA.

When formulating the medicaments usable according to the present invention as suspensions in a preferred embodiment thickening agents to prevent the setting of Pirlindole or a pharmaceutically acceptable salt thereof, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrol, carboxymethyl cellulose, Pluronics^{®} or polyethylene glycol sorbit fatty acid ester. Pirlindole or a pharmaceutically acceptable salt thereof can also be incorporated in liquid formulations in the form of inclusion compounds e.g. with cyclodextrins. In particular embodiments dispersing agents can be added as further adjuvants. For the production of lyophilisates scaffolding agents like mannite, dextran, saccharose, human albumin, lactose, PVP or varieties of gelatine can be used.

In as far as Pirlindole is not included in a liquid drug formulation in its basic form it can be employed within the parenterals in the form of its acid addition salt solvates.

A further important systemic application formulation is peroral administration in the form of tablets, hard or soft gelatine capsules, coated tablets, powders, pellets, microcapsules, compressed oblongs, granulates, cachets, lozenges, chewing gum or sachets. These solid perorally administered formulations can also be formulated as retard and depot systems, respectively. Comprised therein are medicaments with a content of one or more micronized active agents, diffusion and erosion forms based on matrix, e.g. by using fats, waxy or polymeric substances or so called reservoir systems. If the medicament is formulated to release Pirlindole over a prolonged period of time retarding agents and agents for the controlled release, respectively, can be added like film or matrix forming substances, for example, ethylcellulose, hydroxypropyl methyl cellulose, poly(meth)acrylate derivatives, (e.g. Eurdragit^{®}, hydroxypropyl-methylcellulose phthalate both in organic solutions and in the form of aqueous dispersions. In this context bioadhesive preparations should also be mentioned wherein an extended dwelling time in the body is caused by the intimate contact with the mucous membranes of the body. An example of a bioadhesive polymere is, e.g. the group of Carbomere^{®}.

For the purpose of a controlled release of Pirlindole or a pharmaceutically acceptable salt thereof within the different segments of the gastro-intestinal tract it is possible to employ a mixture of pellets which release at different locations. The medicament formulation can be coated, for example, with mixtures of films, substances, compounds or compositions soluble in gastric juice and resistant to gastric juice, respectively. The same purpose of affecting the release in different sections of the gastro-intestinal tract can also be reached with appropriately produced coated tablets with a core, wherein the coating releases the active ingredient in gastric juice rapidly and the core releases the active ingredient in the environment of the small intestine. The aim of a controlled release in different sections of the gastro-intestinal tract can also be achieved by multiple coated tablets. Mixtures of pellets with differentially releasable active agent can be filled into, for example, hard gelatine capsules.

A further adjuvant employed in the production of compressed formulations like e.g. tablets, hard and soft gelatine capsules as well as coated tablets and granules are, for example, counter glue agents, lubricating agents and separating agents, dispersion agents like e.g. flame dispersion silicon dioxide, disintegrants like, e.g. various types of starch, PVP, cellulose, ester as granulating or retarding agent like, e.g. waxy and/or polymeric substances based on Eudragit^{®}, cellulose or Cremophor^{®}.

Furthermore medicaments formulated for peroral administration can comprise antioxidants, sweetening agents like, e.g. saccharose, xylite or mannite, taste correcting agents, flavorants, preservatives, colouring agents, buffering agents, direct compression excipients, microcrystalline cellulose, starch, hydrolyzed starch (e.g. Celutab^{®}), lactose, polyethylene glycol, polyvinylpyrrolidone, dicalcium phosphate, lubricants, fillers like, e.g. lactose or starch, binders in the form of lactose, types of starch like e.g. wheat or corn and rice starch, respectively, derivatives of cellulose like, e.g. methyl cellulose, hydroxypropyl cellulose or silica, talcum, stearate like, e.g. magnesium stearate, calcium stearate, talk, siliconized talk, stearic acid, cetyl alcohol or hydrogenated fats etc. A variety of substances are known to someone of skill in the art which can be added to medicaments for the formulation for peroral administration.

In a further embodiment Pirlindole or a therapeutically acceptable salt thereof can also be formulated as an oral therapeutic system, in particular based on osmotic principles like, e.g. GIT (gastro-intestinal therapeutic system) or OROS (oral osmotic system).

Effervescent tablets or tabs are also among compressed formulations, which can be perorally administered and which are both rapidly dissolvable or suspendable in water and are rapidly drinkable instant drug formulations.

Perorally administrated formulations also include solutions e.g. drops, juices and suspension which can be produced according to methods known in the art and which can comprise - beside the already mentioned adjuvants and additives for the increase of the stability - preservatives and if desired flavouring agents for easier ingestion and colouring agents for better distinction as well as antioxidants and/or vitamins and sweetening agents like sugars or artificial sweeteners. This also applies to dried juices which are prepared with water prior to use. In a preferred embodiment of a formulation of the medicaments of the present invention an ingestible liquid formulation can also comprise an ion exchange resin.

A special release formulation is the construction of so called floating drug formulations, for example, on the basis of tablets or pellets which produce gases after contact with bodily fluids and which, therefore, float on the surface of gastric juice. Furthermore it is also possible to formulate so called electronically directed release systems wherein the release of the active ingredient can be adjusted to the individual requirements by external electronic impulses.

Rectally applicable medicaments are a further group of drug formulations, which can be systemically administered and if desired can also be topically effective. Among those are suppositories and clyster formulations. Clyster formulations can be prepared on the basis of tablets together with aqueous solvents for the production of this administration. It is also possible to provide rectal capsule formulations on the basis of, for example, gelatine or other carriers known in the art.

As basis for suppositories one can consider hard fats like, e.g. Witepsol^{®}, Massa Estarium^{®}, Novata^{®}, coconut oil, glycerine/gelatine matters, glycerine/soaps-gels and polyethylene glycols.

For long term application with a systemic release of active agent over a period of up to several weeks compressed implants are suitable, which are preferably formulated on the basis of so called biodegradable polymers.

The medicament comprising Pirlindole or a pharmaceutically acceptable salt thereof formulated according to the invention can also be formulated as a transdermal system. This formulation just like the above-mentioned rectal form is characterized by circumventing the liver circulation and liver metabolism, respectively. Particularly suitable as transdermal systems are band-aids on the basis of different layers and/or mixtures of suitable adjuvants and carriers, which are capable of releasing the active ingredient in a directed manner over longer or shorter period of time. During the manufacturing of such transdermal systems substances can be added for improving and/or accelerating the penetration of the skin which increase the membrane penetration and as the case may be permeation promoters like, e.g. oleic acid, Azone®, adipic acid derivatives, ethanol, urea, propylene glycol. Beside suitable adjuvants and carriers, solvents, polymeric components, e.g. on the basis of Eudragit®, can be considered as further components of the medicament usable according to the present invention.

Moreover, transdermal delivery is also suitable for skin disorders curable by topical administration of Pirlindole or a pharmaceutical acceptable salt which also effect other organs than the skin. In case of psoriasis, the disease often not only affects the skin, but inflammation affects also the joints resulting in psoriatic arthritis. Thus, topical administration of Pirlindole or a pharmaceutical acceptable salt of a patient having psoriasis can lead to the treatment of both the skin symptoms and the inflammation affecting other parts of the body.

Thus, topical administration of Pirlindole or a pharmaceutical acceptable salt is especially suitable for such skin disorders, especially psoriasis.

Moreover, the invention relates to a topical medicament comprising Pirlindole or a pharmaceutical acceptable salt thereof.

In a more preferred embodiment, the topical medicament is characterized in that it consists of Pirlindole or a pharmaceutical acceptable salt thereof formulated in an oil-in water or water-in oil emulsion. Preferred topical formulations are an emulsion, a gel, an ointment, a foam, a band-aid, a cream of a mixed-phase and amphiphilic, respectively, emulsion system (oil/water-water/oil-mixed-phase), a liposome or transferosome. A particular preferred formulation comprises basis cream DAC (DAC Basiscreme).

Basis cream DAC (DAC Basiscreme) is a cream formulation for topical use. 100 g of the cream have following composition:

| | |
|---|---|
| Glycerol monostearate 60 | 4.0 g |
| Cetyl alcohol | 6.0 g |
| Middle chain triglycerides | 7.5 g |
| White Vaseline | 25.5 g |
| Macrogol-20-glycerolmonostearate | 7.0 g |
| Propylene glycol | 10.0 g |
| Aqua purificata | 40.0 g |

In a preferred embodiment the topical medicament contains between 0.01%-10% Pirlindole or a pharmaceutical acceptable salt thereof based on the weight of the total formulation, preferably between 0.1%-8% Pirlindole or a pharmaceutical acceptable salt thereof, even more preferred between 1% and 4% Pirlindole or a pharmaceutical acceptable salt thereof. It is particular preferred that these amounts of Pirlindole or of a pharmaceutically acceptable salt thereof are comprised in the preferred or particular preferred topical formulations as outlined above.

The invention also relates to pirlindole for use in the treatment of a disease selected from the group consisting of psoriasis, atopic dermatitis, alopecia areata, alopecia totalis, alopecia subtotalis, alopecia universalis, alopecia diffusa, lupus erythematodes of the skin, lichen planus, dermatomyostis of the skin, atopic eczema, morphea, sklerodermia, psoriasis vulgaris, psoriasis capitis, psoriasis guttata, psoriasis inversa, alopecia areata ophiasis-type, androgenetic alopecia, allergic contact eczema, irritative contact eczema, contact eczema, pemphigus vulgaris, pemphigus foliaceus, pemphigus vegetans, scarring mucosal pemphigoid, bullous pemphgoid, mucous pemphigoid, dermatitis, dermatitis herpetiformis duhring, urticaria, necrobiosis lipoidica, erythema nodosum, lichen vidal, prurigo simplex, prurigo nodularis, prurigo acuta, linear IgA dermatosis, polymorphic light dermatoses, erythema solaris, lichen sclerosus et atrophicans, exanthema of the skin, drug exanthema, purpura chronica progressiva, dihidrotic eczema, Eczema, fixed drug exanthema, photoallergic skin reaction, lichen simplex eriorale, dermatitis, acne, rosacea, abnormal scarring, keloids and vitiligo in which a topical medicament according to the invention is administered by application onto the skin.

In an especially preferred embodiment, the disease is selected from psoriasis and atopic dermatitis, especially psoriasis.

The drug formulations suitable for the respective mode of administration can be produced by someone of skill in the art in accordance with formulation instructions and modes of operation on the basis of generally known pharmaceutical-physical concepts.

### Combination with further substances

In a further embodiment of the present invention Pirlindole or a pharmaceutically acceptable salt thereof can be combined with other therapeutically active ingredients which are suitable for the treatment and/or prevention of diseases characterized by hyperproliferation of keratinocytes and/or T cells.

Thus, the present invention relates in a further aspect to compositions comprising Pirlindole or a pharmaceutically acceptable salt thereof and one or more further active ingredients known to be usable for the therapy or prevention of diseases characterized by hyperproliferation of keratinocytes and/or T cells. Particularly suitable active ingredients, which can be combined with Pirlindole or a pharmaceutically acceptable salt thereof are vitamin D derivatives which are agonists of vitamin D receptors, in particular Calcipotriol, retinoids which are agonist of retinoid receptors (RAR), for example, tazarotene, corticosteroid derivatives which are agonist of glucocorticoids, for example, betamethasone and cortisone, fumaric acid, clobetasol, methotrexate amphotericin, busulphane, cotrimoxazole, chlorambucil, colony stimulating factor, cyclophosphamide, fluconazole, ganciclovir, anti-lymphocyte immunoglobulins regular immunoglobulins methylprednisolone, octreotide, oxpentifylline, thalidomide, zolimomab aritox, Clotrimazole.

A further particularly preferred embodiment relates to a composition comprising Pirlindole or a pharmaceutically acceptable salt thereof and a calcineurin antagonist. The term "calcineurin antagonist" within the meaning of the present invention has to be understood to relate to substances that act as antagonists on the calcineurin phosphatase activity. Whether a substance acts antagonistically on calcineurin phosphatase activity can be determined by assays for the determination of calcineurin phosphatase activity described in the prior art. For example, an assay can be carried out as described in Baughman et al. (1995, Mol. Cell. Biol., 15: 4395-4402). The reaction therein comprises 100 µmol/l CaCl₂, 100 µg bovine serum albumin (fraction V) per ml, 40 mmol/l Tris-HCl (pH 8.0), 100 mmol/l NaCl, 6 mmol/l magnesium acetate, 500 µmol/l dithiothreitol, 40 µmol/l [³³P] RII-peptide (600 cpm/pmol), 190 nmol/l bovine calmodulin, 3 nmol/l bovine calcineurin, 50 µmol/l of the substance to be tested ("test substance") for calcineurin inhibition and one immunophilin, e.g. FKBP12 and cyclophilin. The RII-peptide has the sequence DLDVPIPGRFDRRVSVAAE. The phosphorylation at serine residues is carried out as described in Liu et al. (1991, Cell, 66: 807-815) and in Manalan and Klee (1983, PNAS, 87: 4291-4295). The reactions are incubated in the absence of peptide for 30 minutes at 30°C. The dephosphorylation reaction is started by the addition of peptides and then incubated for 10 minutes at 30°C. The termination of the reaction as well as the separation of the free phosphates from phosphorylated peptides is carried out as described in Liu *et al*. and Manalan and Klee (supra). The degree of dephosphorylation measured in the absence of test substance is defined as 100% calcineurin activity while the degree of dephosphorylation measured in the absence of test substance and calcineurin is defined as 0% calcineurin activity. The activity of the respective calcineurin antagonist can then be expressed as a percentage of the decrease of the calcineurin activity in the presence of the respective antagonist. The calcineurin antagonists which are used in compositions of the present invention decrease the calcineurin activity by at least about 10% preferably by at least about 30%, more preferable by at least about 50% and most preferably by at least about 90%. Calcineurin antagonists according to the invention are known from, for example, WO 95/040461, WO 90/14826, EP 0 378 321, WO 95/09857, WO 96/35299, EP 0 626 385, GB 1491509 and DE 2941080.

Preferably the composition according to the present invention comprises one or more calcineurin antagonists selected from cyclosporine A, cyclosporine G, cyclosporine B, cyclosporine C, cyclosporine D, dihydro-cyclosporine D, cyclosporine E, cyclosporine F, cyclosporine H, cyclosporine I, ASM-240, pimecrolimus, tacrolimus, 13-desmethyl derivatives of tacrolimus (L-685487), L-683519 and/or 17-ethyl derivatives of tacrolimus. Particularly preferred are compositions which comprise beside Pirlindole or a pharmaceutically acceptable salt thereof pimecrolimus, tacrolimus and cyclosporine A. In a further preferred embodiment the compositions can comprise one or more of the above-mentioned active ingredients and, thereby in particular one or more of the particularly suitable active ingredients.

The compositions according to the present invention comprising one or more further active ingredients which decrease or inhibit hyperproliferation of keratinocytes and/or T cells and/or one or more calcineurin antagonist can be produced by someone of skill in the art in one of the formulations disclosed above for Pirlindole and can be mixed with respectively indicated adjuvants and additives.

Therefore, a further aspect of the present invention is the use of one of the above-mentioned compositions for the production of a medicament for the therapy or prevention of diseases characterized by hyperproliferation of keratinocytes and/or T cells, in particular atopic dermatitis and psoriasis. During the use according to the present invention of the compositions according to the present invention the same forms of applications as described above for Pirlindole are appropriate in particular the topical application onto affected areas of the skin.

In a further aspect the invention also relates to the spatially and/or temporally separated administration of the respective active ingredients, i.e. Pirlindole, calcineurin antagonist(s) and or active ingredient(s) which decrease the hyperproliferation of keratinocytes and/or T cells.

### Dose

The dose to be applied depends on the respective disease and severity of the respective disease and lies within the discretion of the attending physician. Medicaments usable according to the invention comprise preferably between about 0.01 to about 500 mg active ingredient per dose, preferably between about 1 to about 100 mg active ingredient per dose. The active ingredient can be administered in one or several doses per day; alternatively the active ingredient can be administered in larger time intervals.

In the case of an *in vitro* measurement (examples 2, 3) an inhibitory effect of Pirlindole on the proliferation was already measured at a concentration of Pirlindole of 10 µmol/l. Depending on the permeability of the skin, the type and the severity of the disease and dependent on the type of formulation and frequency of application different concentrations of active ingredients within the medicament can be sufficient to elicit a therapeutic effect by topical application preferably the concentration of Pirlindole or a pharmaceutically acceptable salt thereof within a medicament usable according to the invention is in the range of between 1 µmol/l and 100 mmol/l.

Therefore, in a further embodiment of the present invention a medicament usable according to the invention in particular for topical application is characterized by comprising Pirlindole or a pharmaceutical acceptable salt thereof in a concentration of between 1 µmol/l and 100 mmol/l, preferably between 0.01%-10% Pirlindole, preferably between 0.1%-8% Pirlindole, even more preferred between 1% and 4% Pirlindole (expressed as weight/weight).

The following examples and figures are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice.

### Figures

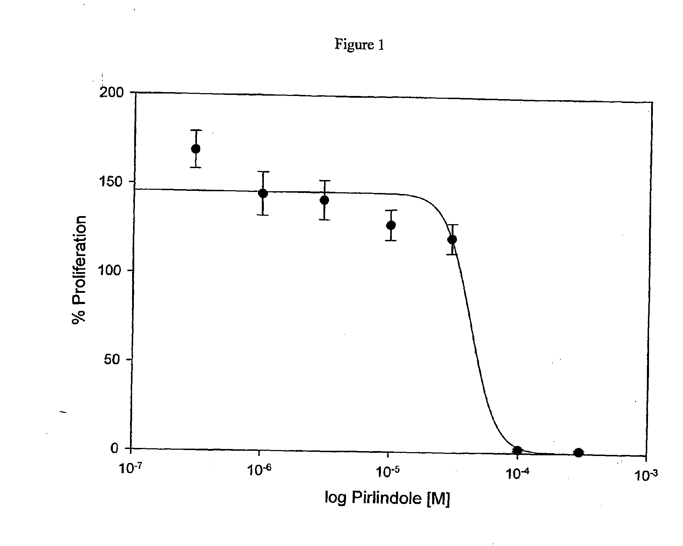

Fig. 1: Effect of Pirlindole on the proliferation of keratinocytes. HaCaT keratinocytes were treated with different Pirlindole concentrations (0.3 µmol/1, 1 µmol/l, 3 µmol/l, 10 µmol/l, 30 µmol/l, 100 µmol/l, 300 µmol/l). At a concentration of 47 µmol/l Pirlindole caused a 50% inhibition of the proliferation in comparison to the positive control (KBM + 10% FCS). As a negative control cells were incubated with KBM. Figure 1 shows one representative experiment.

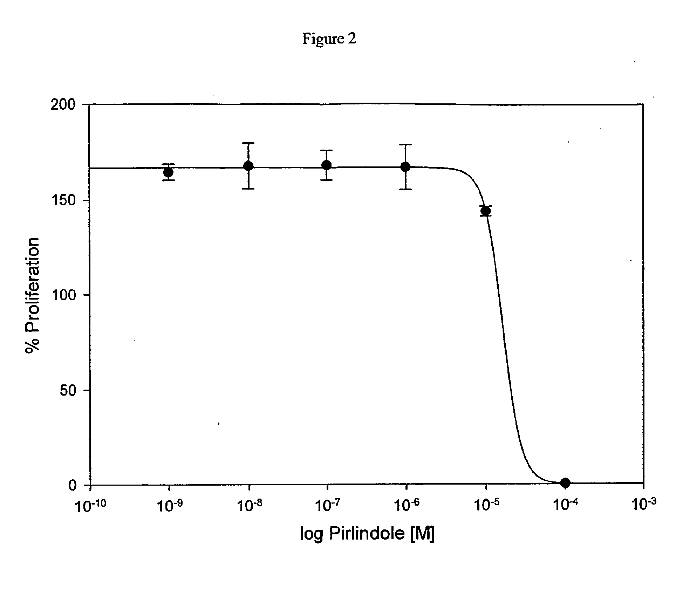

Fig. 2: Effect of Pirlindole on the proliferation of T cells. Peripheral blood mononuclear cells were treated with different Pirlindole concentrations (1 nmol/l, 10 nmol/l, 100 nmol/l, 1 µmol/l, 10 µmol/l, 100 µmol/l). At a concentration of 16 µmol/l the proliferation of α-CD3 stimulated peripheral blood mononuclear cells was inhibited by 50% compared to the proliferation of the positive control (RPMI +10%FCS+ αCD3 antibody). Figure 2 shows one representative experiment.

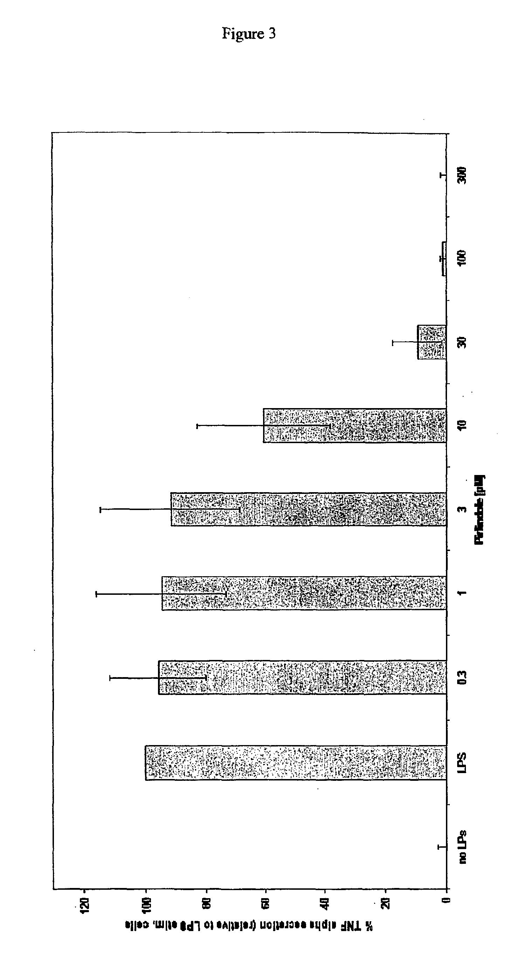

Fig. 3: Effect of Pirlindole on the release of TNFα. The monocytic cell line THP-1 was incubated with different concentrations of Pirlindole (300 µmol/l, 100 µmol/l, 30 µmol/l, 10 µmol/l, 3 µmol/l, 3 µmol/l, 0.3 µmol/l). After stimulation with 50 ng/ml LPS the release of TNFα was determined. Figure 3 shows the means of three independent measurements.

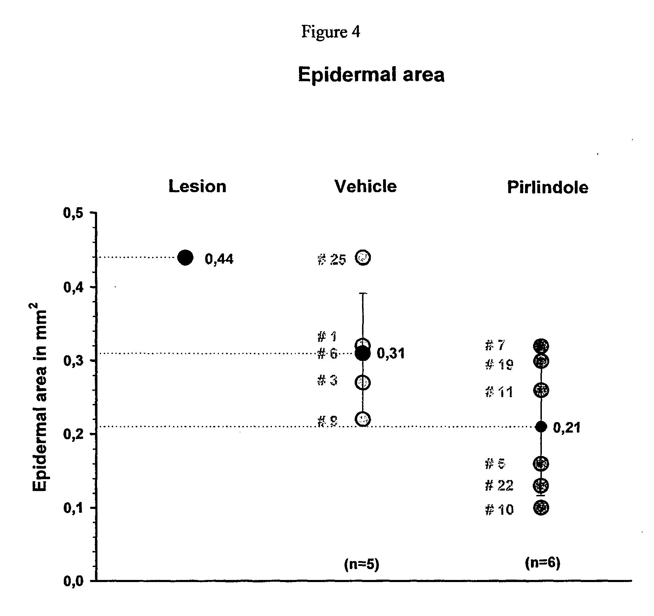

Fig. 4: Effect of Pirlindole on transplanted human psoriatic skin biopsies. SCID mice were transplanted with human psoriatic skin biopsies. Two weeks after surgery therapy started. The mice were treated with daily intraperitonal injections of Pirlindole (50 mg/kg) or vehicle alone. After four weeks of therapy the mice were sacrificed and biopsies were taken. 4 µm slices of the biopsies were stained with Masson trichrom. Epidermal areas were calculated.

### Examples

### Production of Pirlindole

The production of Pirlindole is described in the prior art. Pirlindole can, for example, be produced as described in Gazengel, Jean Marie; Lancelot, Jean Charles; Rault, Sylvain; Robba, Max, Journal of Heterocyclic Chemistry (1990), 27(7), 1947-51.

### Example 1: Influence of Pirlindole on proliferation of keratinocytes

The influence of Pirlindole on proliferation of keratinocytes was examined on the basis of HaCaT cells. For this purpose 5 x 10³ HaCaT keratinocytes were seeded into 60 wells of a 96 well-plate in 200 µl KBM/10% FCS each and incubated for 24 hours at 37°C. After incubation each of 6 wells with HaCaT cells and 1 well without cells were treated for 48 hours with negative control (KBM/1% DMSO), positive control (KBM/FCS/1% DMSO) or with 0,3-300 µmol/l Pirlindole in KBM/FCS (stock solution of Pirlindole: 100 mmol/l in DMSO) and incubated for 48 hours at 37°C. The concentration of DMSO was kept constant at 1% at all tested Pirlindole concentrations. At the end of the second incubation period the medium was removed and cell proliferation was determined by BrDU incorporation (Roche, #1 669 915) according to the manufacturer's instructions. To determine the IC₅₀ of Pirlindole the relative chemiluminescence value of FCS stimulated cells was set to represent 100% proliferation. All other values were divided by the 100% value to obtain the relative percentage of proliferation. The percentage values were used to determine a idealized curve for Pirlindole from which the IC₅₀ was derived (four parameter logistic curve, Sigma Plot). At least four independent experiments were performed. The result of one representative experiment is shown in figure 1. The mean IC₅₀ of Pirlindole is 47 µmol/l. The effect of Pirlindole on the proliferation of keratinocytes shows the particular suitability of Pirlindole for the therapy or prevention of diseases characterized by hyperproliferation of keratinocytes, in particular for the therapy of psoriasis.

### Example 2: Influence of Pirlindole on proliferation of T cells

Peripheral blood mononuclear cells (PBMCs) were isolated by Ficoll-gradient centrifugation from peripheral blood. 1 x 10⁶ PBMCs/ml were re-suspended in RPMI/10% fetal calf serum (FCS) in 96-well plates in a concentration of 2 x 10⁵ cells/well. The cells were incubated with 1 nmol/l, 10 nmol/l, 100 nmol/l, 1µmol/l, 10 µmol/l and 100 µmol/l Pirlindole and stimulated with 10 µg/ml soluble anti-CD3-antibody. As positive and negative controls PBMCs were used, which were stimulated by anti-CD3-antibody and non-stimulated PBMCs, respectively. The final concentration of the solvent DMSO was 0,1% in all examined wells. After two further days of incubation the cells were incubated with 1 µCi per well [³H]-thymidine for 18 hours. The cells were then recovered on glass fibre filters by using a Micro 96 Harvester (Skatron Instruments, Lier, Norway). The incorporated radioactivity was analysed with a Packard Matrix 9600 Counter (Canberra Packard, Schwadorf, Austria). The experiments were carried out with the blood of three different donors. To determine the IC₅₀ of Pirlindole the value of anti-CD3-stimulated cells plus 0,1% DMSO was set to represent 100% proliferation. All other values were divided by the 100% value to obtain the relative percentage of proliferation. The percentage values obtained were used to determine a idealized curve for Pirlindole from which the IC₅₀ was derived (four parameter logistic curve; Sigma Plot). The mean IC₅₀ of Pirlindole is 16 µmol/l. The result of one representative experiment is shown in figure 2. The observation that Pirlindole inhibits the proliferation of activated T cells demonstrates the potency of Pirlindole for the treatment of inflammatory diseases characterised by an increased proliferation of T cells. Since psoriatic and dermatitis lesions are characterised by a strong infiltrate of T lymphocytes and a block of T cell activation is an established therapeutic principle in psoriasis and atopic dermatitis, Pirlindole seems to be particularly useful for the treatment of psoriasis and atopic dermatitis.

### Example 3: Influence of Pirlindole on the secretion of TNFα of LPS stimulated THP-1 cells:

THP-1 cells (2.5 x 10⁴ /well) were seeded in 24 well plates (500 µl RPMI/10%FCS per well) and subsequently treated with Pirlindole (300 µmol/l, 100 µmol/l, 30 µmol/l, 10 µmol/l, 3 µmol/l, 1 µmol/l, 0.3 µmol/l). After 2h cells were stimulated with 50 ng/ml LPS. Six hours after LPS addition, culture supernatants were collected. TNFα concentrations were measured using an enzyme linked immunosorbent assay according to manufacturers protocol (R&D systems, #DTA00C,). The TNFα concentrations reached after LPS stimulation without further treatment was set to 100%. The TNFα values determined after Pirlindole treatment were calculated relative to the 100% value. Concentration of 10 µmol/l and higher caused a clear inhibition of the release of TNFα. TNFα is a validated target for the treatment of psoriasis since a couple a therapeutics aiming the TNFα pathway showed efficacy in development and on the market. The fact Pirlindole is able to reduce TNFα secretion of THP-1 cells strongly supports the suitability of the use of Pirlindole as anti-psoriasis treatment.

### Example 4: Influence of Pirlindole on the psoriatic phenotype in a psoriasis animal model

The effect of Pirlindole on the phenotype of psoriatic skin was determined in an human transplant SCID mouse animal model. (Boehncke et al., Arch. Dermatol. Res., 1994, 286:325-330). Skin biopsies with a spindle shape were taken from the lesion of one psoriasis patient and transplants with a diameter of 0,8 cm were transplanted onto wounds of similar size on the backs of SCID mice. Treatment started two weeks after the transplantation. Pirlindole was dissolved in 4.6% Mannitol/H₂O, pH 6.5. For therapy, 300 µl of a 5 mg/ml solution was daily injected intraperitonally (=50 mg/kg). The control group was injected in the same way with 300 µl vehicle. After four weeks the animals were sacrificed. The biopsies were removed, histologically stained (Masson trichrom) and examined for changes of the epidermal area. The epidermal area of the transplants of the treated animals was clearly decreased in comparison to control animals proving the suitability of Pirlindole for the treatment of hyperproliferative skin diseases like psoriasis.

### Example 5: Influence of Pirlindole on the inflammatory phenotype in a DTH animal model

To test the anti-inflammatory potency of Pirlindole, a delayed-type hypersensitivity ("DTH") reaction is induced at the mouse ear via topical application of a hapten (oxazolone ("OXA") as described by Geba et al., Immunology 104:235-242, 2001; Gaspari & Katz, Current Protocols in Immunology. 1991, 4.2.1-4.2.5. After a challenge an epicutaneous sensibilisation with hapten causes a mainly Th1 type immune reaction (Wang et al., Clin Exp Allergy 29:271-279, 1999). This reaction of the skin with the contact allergen is measured by quantifying the local ear swelling. Various concentrations (4%, 1%, 0.1% and 0.01%) of Pirlindole are analyzed in this experimental set-up. A mixture of acetone/ olive oil (4:1 vol/vol) is used as vehicle.

Ear thickness is measured before start of the treatment (day 0), before challenge (day 6), and 24 h after challenge (d7, ear swelling response) (instrumentation e.g. Mitutoyo - Messgeräte GmbH). In addition, at the end of the experiment ear weights are determined by weighing 8 mm punch biopsies taken from both ears of each individual animal.

It is expected that Pirlindole has led to an improvement of one or more parameters of inflammation severity (ear weight, ear thickness) compared to the placebo control proving the suitability of Pirlindole for the treatment of inflammatory skin diseases like atopic dermatitis and psoriasis.

## Claims

1. Use of Pirlindole or a pharmaceutically acceptable salt thereof if desired with appropriate adjuvants and additives for the production of a medicament for the therapy or prevention of a disease **characterized by** hyperproliferation of keratinocytes and/or T cells.

2. Use according to claim 1, **characterized in that** the disease is selected from psoriasis, atopic dermatitis, actinic keratosis, hyperkeratosis like epidermolytic hyperkeratosis, hyperkeratosis lenticularis perstans, keratosis pilaris and ichthyoses.

3. Use according to claim 1, **characterized in that** the disease is selected from the group consisting of alopecia areata, alopecia totalis, alopecia subtotalis, alopecia universals, alopecia diflusa, atopic dermatitis, lupus erythematodes of the skin, lichen planus, dermatomyositis of the skin, atopic eczema, morphea, scleroderma, psoriasis vulgaris, psoriasis capitis, psoriasis guttata, psoriasis inversa, alopecia areata Ophiasis type, androgenic alopecia, allergic contact dermatitis, irritative contact dermatitis, contact dermatitis, pemphigus vulgaris, pemphigus foliaceus, pemphigus vegetans, scarring mucous membrane pemphigoid, bullous pemphigoid, mucous membrane pemphigoid, dermatitis, dermatitis herpetiformis Duhring, urticaria, necrobiosis lipoidica, erythema nodosum, lichen vidal, prurigo simplex, prurigo nodularis, prurigo acuta, linear IgA dermatosis, polymorphic light dermatosis, erythema Solaris, lichen sclerosus et atrophicans, exanthema of the skin, drug exanthema, purpura chronica progressiva, dihidrotic eczema, eczema, fixed drug exanthema, photoallergic skin reaction, lichen simplex periorale dermatitis, graft-versus-host-disease, acne, rosacea, abnormal scarring, keloids and vitiligo.

4. Use according to one of claims 1 to 3, **characterized in that** the medicament is applied topically.

5. Use according to claim 4, **characterized in that** the medicament is formulated in the form an ointment, a gel, a band-aid, an emulsion, a lotion, a foam, a cream of a mixed phase or an amphiphilic emulsion system (oil/water-water/oil-mixed phase), a liposome, a transferosome, a paste or a powder.

6. Use according to claim 5, **characterized, in that** the cream is cream basis DAC (Deutscher Arzneimittel Codex, DAC Basiscreme),

7. Use according to one of claims 1 to 6, **characterized in that** Pirlindole or a pharmaceutical acceptable salt thereof is comprised within the medicament in a concentration based on the weight of the total formulation of between 0,01%-10% Pirlindole, preferably between 0,1%-8% Pirlindole, even more preferred between 1% and 4% Pirlindole.

8. Use according to one of claims 1 to 7, **characterized in that** Pirlindole or a pharmaceutical acceptable salt thereof is comprised within the medicament in a concentration of between 1 µmol/l and 100 mmol/l.

9. Composition comprising Pirlindole or a pharmaceutical acceptably salt thereof and one or more active ingredients which decrease or inhibit the hyperproliferation of keratinocytes and/or T cells.

10. Composition according to claim 9, **characterized in that** the active ingredient is selected from the group consisting of vitamin D derivates, which are agonists of vitamin D receptors, in particular Calcipotriol, retinoid derivatives which are agonists of retinoid receptors (RAR), in particular tazarotene, corticosteroid derivatives which are agonists of glucocorticoid receptors, in particular betamethasone and cortisone, fumaric acid, clobetasol, methotrexate, amphotericin, busulfan, cotrimoxazole, chlorambucil, colony stimulating factor, cyclophosphamide, fluconazole, ganciclovir, anti-lymphocyte immunoglobulins, regular immunoglobulins, methylprednisolone, octreotide, oxpentifylline, thalidomide, zolimomab aritox and clotrimazole.

11. Composition comprising Pirlindole or a pharmaceutical acceptably salt thereof and one or more calcineurin antagonist(s) selected from the group consisting of cyclosporine A, cyclosporins G, cyclosporine B, cyclosporine C, cyclosporine D, dihydro-cyclosporine D, Cyclosporine E, cyclosporine F, cyclosporine H, cyclosporine I, ASM-240, pimecrolimus, tacrolimus, 13-desmethyl derivatives of tacrolimus and 17-ethyl-derivatives of tacrolimus.

12. Use of a composition according to one of claims 9-11, if needed with suitable adjuvants and additives for the production of a medicament for the therapy or the prevention of diseases **characterized by** hyperproliferation of keratinocytes and/or T cells.

## Patentansprüche

1. Verwendung von Pirlindol oder eines pharmazeutisch verträglichen Salzes desselben, falls gewünscht mit geeigneten Hilfsstoffen und Zusatzstoffen, zur Herstellung eines Medikaments zur Therapie einer Krankheit oder zur Vorbeugung gegen eine Krankheit, die durch Hyperproliferation von Keratinozyten und/oder T-Zellen **gekennzeichnet** ist.

2. Verwendung gemäß Anspruch 1, **dadurch gekennzeichnet, dass** die Krankheit ausgewählt ist aus Psoriasis, atopischer Dermatitis, aktinischer Keratose, Hyperkeratose wie epidermolytischer Hyperkeratose, Hyperkeratosis lenticularis perstans, Keratosis pilaris und Ichthyosen.

3. Verwendung gemäß Anspruch 1, **dadurch gekennzeichnet, dass** die Krankheit ausgewählt ist aus der Gruppe bestehend aus: Alopecia areata, Alopecia totalis, Alopecia subtotalis, Alopecia universalis, Alopecia diffusa, atopischer Dermatitis, Lupus erythematodes der Haut, Lichen planus, Dermatomyositis der Haut, atopisches Ekzem, Morphea, Skleroderma, Psoriasis vulgaris, Psoriasis capitis, Psoriasis guttata, Psoriasis inversa, Alopecia areata vom Ophiasis-Typ, Alopecia androgenetica, allergische Kontaktdermatitis, irritative Kontaktdermatitis, Kontaktdermatitis, Pemphigus vulgaris, Pemphigus foliaceus, Pemphigus vegetans, vernarbendes Schleimhautpemphigoid, bullöses Pemphigoid, Schleimhautpemphigoid, Dermatitis, Dermatitis herpetiformis Duhring, Urticaria, Necrobiosis lipoidica, Erythema nodosum, Lichen vidal, Prurigo simplex, Prurigo nodularis, Prurigo acuta, lineare IgA-Dermatose, polymorphe Lichtdermatose, Erythema solaris, Lichen sklerosus und atrophicans, Exanthem der Haut, Arzneimittel-Exanthem, Purpura chronica progressiva, dihydrotisches Ekzem, fixes Arzneimittel-Exanthem, photoallergische Hautreaktion, Lichen simplex periorale dermatitis, Transplantat-gegen-Wirt-Reaktion, Akne, Rosacea, abnormale Narbenbildung, Keloide, und Vitiligo.

4. Verwendung gemäß einem der Ansprüche 1 bis 3, **dadurch gekennzeichnet, dass** das Medikament topisch verabreicht wird.

5. Verwendung gemäß Anspruch 4, **dadurch gekennzeichnet, dass** das Medikament in Form einer Salbe, eines Gels, eines Heftpflasters, einer Emulsion, einer Lotion, eines Schaums, einer Creme aus einer gemischten Phase oder einem amphiphilen Emulsionssystem (Öl/Wasser-Wasser/Öl-gemischte Phase), eines Liposoms, eines Transferosoms, einer Paste oder eines Puders formuliert ist.

6. Verwendung gemäß Anspruch 5, **dadurch gekennzeichnet, dass** die Creme DAC-Basiscreme ist (Deutscher Arzneimittel Codex, DAC-Basiscreme).

7. Verwendung gemäß einem der Ansprüche 1 bis 6, **dadurch gekennzeichnet, dass** Pirlindol oder ein pharmazeutisch verträgliches Salz desselben in dem Medikament in einer Konzentration zwischen 0,01% bis 10% Pirlindol, vorzugsweise zwischen 0,1% bis 8% Pirlindol, noch mehr bevorzugt zwischen 1% und 4% Pirlindol, basierend auf dem Gewicht der gesamten Formulierung enthalten ist.

8. Verwendung gemäß einem der Ansprüche 1 bis 7, **dadurch gekennzeichnet, dass** Pirlindol oder ein pharmazeutisch verträgliches Salz desselben in dem Medikament in einer Konzentration zwischen 1 µmol/l und 100 mmol/l enthalten ist.

9. Zusammensetzung umfassend Pirlindol oder ein pharmazeutisch verträgliches Salz desselben und einen oder mehrere wirksame Bestandteile, die die Hyperproliferation von Keratinozyten und/oder T-Zellen vermindern oder inhibieren.

10. Zusammensetzung gemäß Anspruch 9, **dadurch gekennzeichnet, dass** der wirksame Bestandteil ausgewählt ist aus der Gruppe bestehend aus Vitamin D-Derivaten, die Agonisten von Vitamin D-Rezeptoren sind, insbesondere Calcipotriol, Retinoid-Derivaten, die Agonisten von Retinoid-Rezeptoren (RAR) sind, insbesondere Tazaroten, Corticosteroid-Derivaten, die Agonisten von Glucocorticoid-Rezeptoren sind, insbesondere Betamethason und Cortison, Fumarsäure, Clobetasol, Methotrexat, Amphotericin, Busulfan, Cotrimoxazol, Chlorambucil, Kolonie-stimulierendem Faktor, Cyclophosphamid, Fluconazol, Ganciclovir, Anti-Lymphozyten-Immunglobulinen, regulären Immunglobulinen, Methylprednisolon, Octreotid, Oxpentifyllin, Thalidomid, Zolimomab aritox und Clotrimazol.

11. Zusammensetzung umfassend Pirlindol oder ein pharmazeutisch verträgliches Salz desselben und einen oder mehrere Calcineurin-Antagonisten, ausgewählt aus der Gruppe bestehend aus Cyclosporin A, Cyclosporinen G, Cyclosporin B, Cyclosporin C, Cyclosporin D, Dihydrocyclosporin D, Cyclosporin E, Cyclosporin F, Cyclosporin H, Cyclosporin I, ASM-240, Pimecrolimus, Tacrolimus, 13-Desmethyl-Derivaten von Tacrolimus und 17-Ethyl-Derivaten von Tacrolimus.

12. Verwendung einer Zusammensetzung gemäß einem der Ansprüche 9 bis 11, falls nötig mit geeigneten Hilfsstoffen und Zusatzstoffen, zur Herstellung eines Medikaments für die Therapie von Krankheiten oder die Vorbeugung gegen Krankheiten, die durch Hyperproliferation von Keratinozyten und/oder T-Zellen **gekennzeichnet** sind.

## Revendications

1. Utilisation de Pirlindole ou d'un sel pharmaceutiquement acceptable de celui-ci, en option avec des adjuvants et des additifs appropriés pour la production d'un médicament pour la thérapie ou la prévention d'une maladie **caractérisée par** une hyperprolifération des kératinocytes et/ ou des cellules T.

2. Utilisation selon la revendication 1, **caractérisée en ce que** la maladie est choisie parmi psoriasis, dermatite atopique, kératose actinique, hyperkératose telle que l'hyperkératose épidermolytique, hyperkératose lenticularis perstans, kératose pilaire et ichtyose.

3. Utilisation selon la revendication 1, **caractérisée en ce que** la maladie est choisie dans le groupe consistant en: pelade, alopécie totale, alopécie sous-totale, alopécie universelle, alopécie diffuse, dermatite atopique, lupus érythémateux de la peau, lichen plan, dermatomyosite de la peau, eczéma atopique, morphée, sclérodermie, psoriasis vulgaire, psoriasis capitis, psoriasis en gouttes, psoriasis interverti, pelade ophiasique, alopécie androgénique, dermatite de contact allergique, dermatite de contact irritative, dermatite de contact, pemphigus vulgaire, pemphigus foliacé, pemphigus végétant, pemphigoïde des membranes muqueuses cicatrisantes, pemphigoïde bulleuse, pemphigoïde des membranes muqeuses, dermatite, dermatite herpétiforme de Duhring, urticaire, nécrobiose lipoïdique,
érythème noueux, lichen Vidal, prurigo simplex, prurigo nodulaire, prurigo aigu, dermatose à IgA linéaire, photodermatose polymorphe, érythème solaire, lichen scléreux et atrophique, exanthème de la peau, exanthème médicamenteux, purpura chronique progressive, eczéma dihidrotique, eczéma, exanthème médicamenteux fixé, réaction cutanée photo-allergique, dermatite périorale à lichen simplex, syndrome de réaction du greffon contre l'hôte, acné, acné rosacée, cicatrisation anormale, chéloïdes et vitiligo.

4. Utilisation selon l'une quelconque des revendications 1 à 3, **caractérisée en ce que** le médicament est appliqué par voie topique.

5. Utilisation selon la revendication 4, **caractérisée en ce que** le médicament est formulé sous la forme d'une pommade, un gel, un pansement adhésif, une émulsion, une lotion, une mousse, une crème d'une phase mixte ou d'un système en émulsion amphiphile (phase mixte huile/eau-eau/huile), un liposome, une transférosome, une pâte ou une poudre.

6. Utilisation selon la revendication 5, **caractérisée en ce que** la crème est une base pour crème DAC (Deutscher Arzneimittel Codex, DAC Basiscreme).

7. Utilisation selon l'une quelconque des revendications 1 à 6, **caractérisée en ce que** le Pirlindole ou un sel pharmaceutiquement acceptable de celui-ci est compris dans le médicament en une concentration sur la base du poids de la formulation totale se situant entre 0,01% et 10% de Pirlindole, de préférence entre 0,1% et 8% de Pirlindole, et plus préférablement entre 1 % et 4 % de Pirlindole.

8. Utilisation selon l'une quelconque des revendications 1 à 7, **caractérisée en ce que** le Pirlindole ou un sel pharmaceutiquement acceptable de celui-ci est compris dans le médicament en une concentration se situant entre 1 µmol/l et 100 mmol/l.

9. Composition comprenant du Pirlindole ou un sel pharmaceutiquement acceptable de celui-ci et un ou plusieurs constituants actifs qui abaissent ou inhibent l'hyperprolifération des kératinocytes et/ ou des cellules T.

10. Composition selon la revendication 9, **caractérisée en ce que** le constituant actif est choisi dans le groupe consistant en dérivés de vitamine D, qui sont des agonistes des récepteurs de vitamine D, en particulier Calcipotriol, dérivés de rétinoïde qui sont des agonistes des récepteurs de rétinoïde (RAR), en particulier tazarotène, dérivés de corticostéroïde qui sont des agonistes des récepteurs de glucocorticoïde, en particulier bêtaméthasone et cortisone, acide fumarique, clobétasol, méthotrexate, amphotéricine, busulfan, cotrimoxazole, chlorambucil, facteur de stimulation des colonies, cyclophosphamide, fluconazole, ganciclovir, immunoglobulines antilymphocytes, immunoglobulines ordinaires, méthylprédnisolone, octréotide, oxpentifylline, thalidomide, zolimomab aritox et clotrimazole.

11. Composition comprenant du Pirlindole ou un sel pharmaceutiquement acceptable de celui-ci et un ou plusieurs antagonistes de calcineurine choisis dans le groupe consistant en cyclosporine A, cyclosporines G, cyclosporine B, cyclosporine C, cyclosporine D, dihydro-cyclosporine D, cyclosporine E, cyclosporine F, cyclosporine H, cyclosporine I, ASM-240, pimecrolimus, tacrolimus, dérivés 13-desméthyle de tacrolimus et dérivés 17-éthyle de tacrolimus.

12. Utilisation d'une composition selon l'une quelconque des revendications 9-11, si nécessaire avec des adjuvants et des additifs appropriés, pour la production d'un médicament pour la thérapie ou la prévention de maladies **caractérisées par** une hyperprolifération des kératinocytes et/ ou des cellules T.
